# EUROPEAN PATENT SPECIFICATION

(11) **EP 0 455 808 B1**
(45) Date of publication and mention of the grant of the patent: **25.05.1994**
(21) Application number: 91904406.5
(22) Date of filing: 27.11.1990
(51) Int. Cl.: A23K 1/00

(54) **NEW USE OF GAMMA BUTYROBETAINE FOR PREVENTING DIET INDUCED CARNITINE DEFICIENCY IN DOMESTICATED DOGS AND CATS**
NEUE VERWENDUNG VON GAMMA BUTYROBETAIN ZUR VERMEIDUNG DES DURCH DIÄT VERURSACHTEN CARNITINMANGELS BEI HAUSHUNDEN UND KATZEN
NOUVELLE UTILISATION DE GAMMA BUTYROBETAINE DE PREVENTION D'UNE CARENCE EN CARNITINE INDUITE PAR UN REGIME ALIMENTAIRE CHEZ LES CHIENS ET LES CHATS DOMESTIQUES

(30) Priority: 27.11.1989 US 441110
(43) Date of publication of application: 13.11.1991
(73) Proprietor: SHUG, Austin L., Madison, Wisconsin 53704 (US); KEENE, Bruce W., Raleigh, North Carolina 27607 (US)
(72) Inventor: SHUG, Austin L., Madison, Wisconsin 53704 (US); KEENE, Bruce W., Raleigh, North Carolina 27607 (US)
(74) Representative: SERJEANTS
(86) International application number: PCT/US90/06976
(87) International publication number: WO 91/07880

(56) References cited:
- WO-A-89/10065
- GB-A- 2 091 101
- JOURNAL OF NUTRITION, vol. 113, no. 10, 1983, pages 1906-1913, US; C.J. REBOUCHE: "Effect of dietary carnitine isomers and gamma-butyrobetaine on L-carnitine biosynthesis and metabolism in the rat"

## Description

The invention relates to the field of pet food compositions and more specifically to pet food enriched with L-Carnitine.

L-Carnitine is a quaternary amine that promotes beta-oxidation of long-chain fatty acids by facilitating their transfer across the mitochondrial membrane. L-Carnitine has also been shown to promote oxidation of branched-chain amino acids and the utilization of acetyl-coenzyme A.

In mammalian species, L-Carnitine concentration in cardiac and skeletal muscle is much higher than in serum. In these tissues fatty acids are utilized as a major source of energy. Because of L-Carnitine's central role in transporting fatty acids to the site of oxidation, adequate levels of L-Carnitine are required for normal fatty acid and energy metabolism in mammalian hearts. This is evidenced by the restoration to normal of fatty acid oxidation in muscle homogenates of certain L-Carnitine deficient patients. A relationship between deficient levels of myocardial L-Carnitine and cardiomyopathy has been observed in both hamsters and dogs. Restoration toward normal of such deficient L-Carnitine levels has been shown to result in improved myocardial function in both species.

In mammals, L-Carnitine is derived from the diet and from biosynthesis in the liver, and in some species, kidney and other tissues. Neither cardiac nor skeletal muscle is capable of synthesizing L-Carnitine, however. Thus, the L-Carnitine found in these tissues was either absorbed from the diet or biosynthesized endogenously by other tissues.

Our International Patent Application published as WO89/10065 is directed to a new use, in veterinary medicine for L-Carnitine and proposes a dietary supplement for domesticated dogs and cats, containing a prophylactic amount of L-Carnitine. The invention of WO89/10065 was based on the observation that dogs and cats were unable to maintain a stable and optimum level of L-Carnitine in the same way as other mammalian species studied. The majority of mammalian species reabsorb, in the kidney, L-Carnitine from the urine. Renal reabsorption permits a stable and optimum level of L-Carnitine to be maintained. Dogs and cats, however, did not have the ability of renal absorption to a sufficient degree, so that in the wild they were dependent on diet to replace L-Carnitine excreted in the urine and to maintain a stable and optimum physiological level. Previous researchers had relied the on L-Carnitine levels in domesticated dogs and cats as indicating the stable and optimum physiological levels. The present inventors showed in WO89/10065 that in comparison with the same species in the wild, domesticated dogs and cats tended to suffer from acute L-Carnitine deficiency.

Studies have been made on the effectiveness of the biological precursors of L-Carnitine, ε-N-trimethyl-L-lysine and γ-butyrobetaine, as nutritional additives in the feeding of human babies. (A.L. Olson & C.J. Rebouch, Amer. Inst. of Nutrition, 1987 February.).

γ-butyrobetaine is a biological precursor of L-Carnitine in the biosynthetic pathway of the latter compound. See Nutrition Reviews, Vol. 36, No. 10, pp.305-309, 1978, the teachings of which are incorporated herein by reference. γ-butyrobetaine has been used successfully to alleviate carnitine deficiency syndromes in humans as described in U.S. Patent No. 4,382,092 to Cavazza.

Olson and Rebouche compared L-Carnitine excretion rates in rats and in human infants when fed comparable doses of dietary γ-butyrobetaine and ε-N-trimethyl-L-lysine. They concluded that the γ-butyrobetaine was biologically converted to L-Carnitine in human infants at a rate greater than that at which it was excreted by renal activity. Conversely, with ε-N-trimethyl-L-lysine, the renal excretion was at a rate greater than the conversion rate of γ-N-trimethyl-L-lysine . In the absence of comparative tests on human adults, Olson and Rebouche declined to draw any conclusions on the uniqueness of this pattern of renal behaviour to human infants.

The inability of Olson and Rebouche to predict the renal activity of human adults from studies on human infants demonstrates the impossibility of extrapolating such results to other species, and particularly to species such as dogs and cats which have been shown to exhibit quite unique renal activity in the handling of L-Carnitine itself.

With the above background, the inventors set out to investigate the possibility of using a low-cost biological precursor of L-Carnitine as a dietary supplement for domesticated dogs and cats, to prevent diet-induced carnitine deficiency.

### Summary of the Invention

The invention is based on a study of L-Carnitine levels established and maintained in domesticated dogs and cats fed with a dietary supplement of γ-butyrobetaine, from which study it was deduced that the conversion of γ-butyrobetain into L-Carnitine occurs at a rate greater than removal of the γ-butyrobetaine by the kidneys.

The invention provides a new use for γ-butyrobetaine, for the manufacture of a pet food composition for daily feeding to a dog or cat, containing a prophylactic amount of γ-butyrobetaine. The administration of the γ-butyrobetaine is effective in preventing diet-induced carnitine deficiency in the dog or cat.

The appropriate prophylactic amount of γ-butyrobetaine for incorporation into the pet food is preferably at least 1.0 gram of γ-butyrobetaine per kilogram of mixture and advantageously from 1.0 to 5.0 grams of γ-butyrobetaine per kilogram. The prophylactic amount is preferably sufficient to produce and maintain in the dog or cat a plasma total L-Carnitine concentration of at least 40 µM/liter of plasma.

### Best Mode of Carrying Out the Invention:

Studies were made of the levels of serum L-Carnitine in dogs for a period following oral administration of 5 grams of γ-butyrobetaine. The blood serum level of L-Carnitine was found to rise markedly to a desirably high level within two hours of administration of the γ-butyrobetaine. Moreover, it was observed that the blood serum level of L-Carnitine was maintained at levels close to the initial peak level for at least six hours, indicating that secretion of the γ-butyrobetaine by the kidneys was not at a rate greater than the biological utilization of γ-butyrobetaine.

The results are shown in the Table below.

### TABLE

Changes in serum L-Carnitine following oral administration of 5 grams of gamma-butyrobetaine. Amounts are given in µM/l.

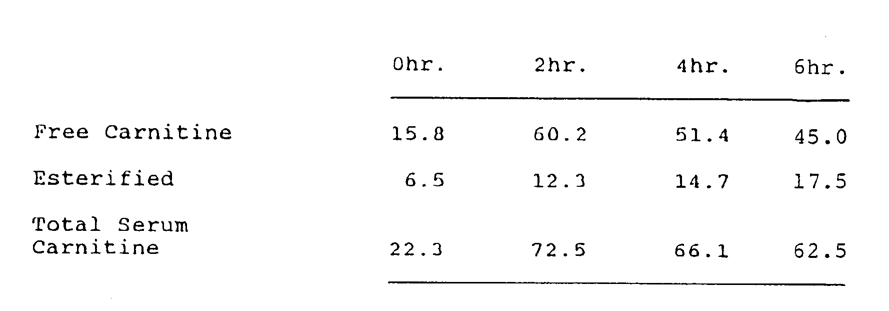

Similar results were indicated from studies on cats.

In the preferred embodiment, sufficient γ-butyrobetaine will be administered orally to the pet dog or cat to raise the serum L-Carnitine level to 40.0 µM/liter. of plasma. Administration can be accomplished in the manner described in WO89/10065 for L-Carnitine, but the amount of γ-butyrobetaine needed for the same amount of rise in serum L-Carnitine will be greater. In practice, between 1 to 5 grams of γ-butyrobetaine should be administered daily, with the preferred amount being between 3 and 4 grams of γ-butyrobetaine.

The use of γ-butyrobetaine in lieu of L-Carnitine as a supplement provides a distinct economic advantage as the industrial preparation of γ-butyrobetaine is less complicated and less expensive than the preparation of L-Carnitine. The L-Carnitine preparation requires the optical antipode resolution of the racemic mixture which is unavoidably obtained in the chemical synthesis of carnitine, and this necessarily increases the complexity and the expense of the synthesis.

## Claims

1. Use of γ-butyrobetaine for the manufacture of a pet food composition for daily feeding to a dog or cat, containing a prophylactic amount of γ-butyrobetaine,

2. Use of γ-butyrobetaine as claimed in claim 1 wherein said γ-butyrobetaine is in an amount from 1.0 to 5.0 grams per kilogram of pet food.

3. Use of γ-butyrobetaine as claimed in claim 2 wherein said γ-butyrobetaine is in an amount of from 3.0 to 4.0 grams per kilogram of pet food.

## Patentansprüche

1. Verwendung von γ-Butyrobetain zur Herstellung einer Haustier-Lebensmittelzusammensetzung, die eine prophylaktische Menge γ-Butyrobetain enthält, zum täglichen Verfüttern an einen Hund oder eine Katze.

2. Verwendung von γ-Butyrobetain nach Anspruch 1, bei der die Menge γ-Butyrobetain 1,0 bis 5,0 g pro Kilogramm Haustiernahrungsmittel beträgt.

3. Verwendung von γ-Butyrobetain nach Anspruch 2, bei der die Menge γ-Butyrobetain zwischen 3,0 und 4,0 g pro Kilogramm Haustiernahrungsmittel beträgt.

## Revendications

1. Utilisation de γ-butyrobétaïne dans la fabrication d'une composition nutritive pour animaux domestiques destinée à l'alimentation quotidienne d'un chien ou d'un chat, contenant une proportion prophylactique de γ-butyrobétaïne.

2. Utilisation de γ-butyrobétaïne selon la revendication 1, dans laquelle ladite γ-butyrobétaïne est présente en une proportion de 1,0 à 5,0 grammes par kilogramme d'aliments pour animaux.

3. Utilisation de γ-butyrobétaïne selon la revendication 2, dans laquelle ladite γ-butyrobétaïne est présente en une proportion de 3,0 à 4,0 grammes par kilogramme d'aliments pour animaux.
